# EUROPEAN PATENT APPLICATION

(11) **EP 4 099 018 A1**
(43) Date of publication of application: **07.12.2022**
(21) Application number: 21382486.5
(22) Date of filing: 31.05.2021
(51) Int. Cl.: G01N 33/569, G01N 33/573, G01N 33/68

(54) **SALIVARY ACTIVITY OF ACE2 AS A MARKER TO PREDICT SUSCEPTIBILITY TO SARS-COV-2 AND COVID19 SEVERITY**

(71) Applicant: Consejo Superior de Investigaciones Científicas (CSIC), 28006 Madrid (ES); Fundación para la Investigación Biomédica del Hospital Universitario Ramón y Cajal, 28034 Madrid (ES)
(72) Inventor: FERRER MARTÍNEZ, Manuel, Madrid (ES); SERRANO VILLAR, Sergio, Madrid (ES)
(74) Representative: Pons

(57) **Abstract**

The invention relates to biomarkers and methods to predict the susceptibility of a subject to be infected by SARS-CoV-2 and to predict the development of severe COVID19 symptoms. Variations in the ACE2 activity in saliva explain the striking differences of susceptibility to infection and risk of severe disease.

## Description

The invention relates to biomarkers and methods to predict the susceptibility of a subject to be infected by SARS-CoV-2 and to predict the development of severe COVID19 symptoms.

### BACKGROUND ART

Because the angiotensin-converting enzyme 2 (ACE2) is the molecular target for severe acute respiratory syndrome coronavirus 2 (SARS-CoV-2) cell entry, variations in the expression and therefore quantity of ACE2 on cells surface may play a significant role in determining the outcome of an individual that comes into contact with SARS-CoV-2 (Choudhary S, et al, Ann. Lab. Med. 2021; 41: 129-38). ACE2 is widely expressed in different tissues, including the surface of cells of the lungs, nasal cavity, intestines, kidney, testis, gallbladder, and heart, explaining why direct person-to-person respiratory transmission is the primary means of SARS-CoV-2 transmission (Meyerowitz EA et al., Ann Intern Med 2021; 174: 69-79).

Reports of pathologic findings in tissue specimens of COVID-19 patients are emerging and reinforce the role of ACE2 expression in disease pathogenesis. ACE2 expression in the lungs seems to increase with age, which might explain the higher disease severity observed in the older population with COVID-19. However, whether ACE2 expression correlates with susceptibility and age remains unclear. While children and women are assumed to have a lower incidence of infection than adult men, the potential role of ACE2 expression in the lungs has not been fully explored (Ciaglia E et al., Front Pediatr 2020; 8).

Due to the rapidly evolving pandemic situation, there is a pressing need for methods to predict both the susceptibility of individuals to be infected by SARS-CoV-2 as well as methods to prognose the severity of COVID19 in individuals already infected. Said methods ideally should be quick and easily performed in samples readily obtained from subjects. The existing methods, such as the one disclosed in Sanjana R. et al. (mSphere 2021 Apr 28;6(2):e00203-21) or other methods mostly based on patient characteristics (reviewed in Gallo Marin et al, Rev Med Virol. 2021 Jan; 31(1): 1-10) are neither quick nor simple and in many cases lack robustness. Thus, there is a need for alternative methods of predicting the susceptibility of individuals to SARS-CoV-2 infection as well as prognosing COVID19 severity.

### DESCRIPTION OF THE INVENTION

The inventors have found that a low activity of angiotensin-converting enzyme 2 (ACE2) in saliva correlates with lower susceptibility to SARS-CoV-2 infection and explains, at least in part, the formidable clinical phenotype of individuals with repeated high-risk exposures to SARS-CoV-2 who did not become infected. Additionally, the inventors also discovered that the level of activity of ACE2 in saliva allows the prediction of the evolution of COVID19.

Therefore, a first aspect of the present invention relates to a use of the level of activity of the angiotensin-converting enzyme 2 (ACE2) biomarker in a saliva sample isolated from a subject for the *in vitro* prediction of coronavirus disease 2019 (COVID19) severity, form now onwards the COVID19 prediction use of the invention.

Another aspect of the present invention is related to a use of the level of activity of the ACE2 biomarker in a saliva sample isolated from a subject for the *in vitro* prediction of susceptibility to infection by severe acute respiratory syndrome coronavirus 2 (SARS-CoV-2), from now onwards the SARS-CoV-2 prediction use of the invention.

The expression "angiotensin-converting enzyme 2" or its acronym "ACE2" as used herein refers to a zinc-containing enzyme which catalyzes the hydrolysis of angiotensin II (a vasoconstrictor peptide) into angiotensin (a vasodilator). ACE2 functions therefore as a carboxypeptidase (EC 3.4.17.23), cleaving a single C-terminal residue from a distinct range of substrates. The expression "level of activity of ACE2" as used herein refers to the capacity of ACE2 to hydrolyze or cleave the C-terminal of an appropriate target substrate. Said appropriate target substrates are characterized by a consensus sequence of Proline-X-(1-3 residues)-Proline-Hydrophobic amino acid, with hydrolysis happening between proline and the hydrophobic amino acid. Despite this consensus sequence, ACE2 has also been showed to be able to hydrolyze synthetic substrates that contain a 3 amino acid sequence of Alanine - Proline - Lysine (APK) (Vickers et al, ENZYME CATALYSIS AND REGULATION, 2002, 277(17), p14838-14843). ACE2 is found attached to the membrane of cells located in the intestines, kidney, testis, gallbladder, and heart. ACE2 is a single-pass type I membrane protein, with its enzymatically active domain exposed on the surface of cells which contain it. The extracellular domain of ACE2 is cleaved from the transmembrane domain by another enzyme known as sheddase, and the resulting soluble protein is released into the intercellular space, bodily fluids and ultimately secreted through urine.

The term "biomarker" as used herein refers to a protein whose activity can be objectively determined and quantified in a saliva sample and the said activity is used both as a predictor of the susceptibility of an individual to be infected by SARS-CoV-2 as well as a predictor for the severity of COVID19 in an individual. In the present invention the biomarker refers to the protein ACE2 (Uniprot accession number: Q9BYF1 sequence version 2). In a preferred embodiment of the COVID19 prediction use or of the SARS-CoV-2 prediction use of the invention, the ACE2 biomarker comprises an amino acid sequence with at least 80%, 85%, 90%, 91%, 92%, 93%, 94%, 95%, 96%, 97%, 98%, 99% or 100% identity to SEQ ID NO:1. In another preferred embodiment of the COVID19 prediction use of the invention or of the SARS-CoV-2 prediction use of the invention, the ACE2 biomarker consists of SEQ ID NO: 1.

SEQ ID NO: 1
















The expression "level of activity of the ACE2 biomarker" as used herein refers to the quantification of the capacity of ACE2 to hydrolyze or catalyze the cleavage of an amino acid fragment in an appropriate target substrate wherein said cleavage is detectable and therefore quantifiable. Said quantification is performed in a "saliva sample" a term which as used herein refers to a part or small quantity of saliva which is considered to be representative of the whole and which is taken or separated therefrom for the purpose of study, analysis or experimentation. Methods to obtained saliva from a subject are known to the expert in the field who can easily determine the best method to apply regarding the requirements of the present invention. In the present invention the saliva sample is obtained from a subject. In preferred embodiment of the COVID19 prediction use of the invention or of the SARS-CoV-2 prediction use of the invention the subject is mammal, preferably a human of any age and gender.

The term "prediction" as used herein refers to the procedure to determine the probability or likelihood of a subject developing a severe case of a certain disease, nosological entity, syndrome, or health-disease condition, or the procedure to determine the probability or likelihood of a subject to be infected or contaminated by a pathogen, such as bacteria, virus, nematode, fungi, which may lead to the development of said disease, nosological entity, syndrome, or health-disease condition. In both cases the procedure the determination is made by analyzing a series of clinical parameters or symptoms characteristic of that disease, or individual characteristics that affect the clinical parameters. In the present invention the predictions made are for "COVID19 severity" and "susceptibility to infection by SARS-CoV-2", and in both cases the clinical or individual parameter is the level of activity of the ACE2 biomarker. As those skilled in the art will understand, such a prediction assessment, although preferred, may not normally be correct for 100% of the subjects to be assessed. The term, however, requires that a statistically significant proportion of the subjects can be identified as developing a severe case of COVID19 or being at increased risk of being infected by SARS-CoV-2. The number that is statistically significant can be established by a person skilled in the art by using different statistical tools, for example, but not limited to, determination of confidence intervals, determination of P-significance value, Student's test or Fisher's discriminant functions, Mann Whitney non-parametric measures, Spearman's correlation, logistic regression, linear regression, area under the ROC curve (AUC). Preferably, the confidence intervals are at least 90%, at least 95%, at least 97%, at least 98% or at least 99%. Preferably, the p-value is less than 0.1, less than 0.05, less than 0.01, less than 0.005 or less than 0.0001. Preferably, the present invention allows to correctly detect the severity of COVID19 or susceptibility of being infected by SARS-CoV-2 differentially in at least 70%, more preferably in at least 80%, much more preferably in at least 90% of the subjects of a given group or population tested.

The term "coronavirus disease 2019" or its acronym "COVID19" as used herein refer to the contagious disease caused by severe acute respiratory syndrome coronavirus 2 (SARS-CoV-2). The level of "COVID19 severity" is variable from individual to individual. At least a third of individuals who are infected do not develop noticeable symptoms, while those who do develop noticeable symptoms enough to be classed as patients, most (81%) develop mild to moderate symptoms, such as, but not limited to, mild pneumonia, while 14% develop severe symptoms, such as, but not limited to, dyspnea, hypoxia, or more than 50% lung involvement on imaging, and 5% suffer critical symptoms, such as, but not limited to, respiratory failure, shock, or multiorgan dysfunction.

The expressions "coronavirus disease 2019 severity" or "COVID19 severity" a used herein refer to the classification of patients with COVID19 into two categories: mild and severe. Mild patients where considerer those individuals who did not require supplemental oxygen and remained asymptomatic after one week of diagnosis. Severe patients where considerer those individuals who presented bilateral radiologic infiltrates or opacities and clinical assessment requiring supplemental oxygen.

The term "severe acute respiratory syndrome coronavirus 2" or its acronym "SARS-CoV-2" as used herein refer to the virus that causes COVID-19, previously also named 2019 novel coronavirus (2019-nCoV), and human coronavirus 2019 (HCoV-19 or hCoV-19). SARS-CoV-2 is a positive-sense single-stranded RNA (+ssRNA) virus, with a single linear RNA segment. SARS-CoV-2 virion is 50-200 nanometres in diameter, which is composed by four structural proteins, known as the S (spike), E (envelope), M (membrane), and N (nucleocapsid) proteins; the N protein holds the RNA genome, and the S, E, and M proteins together create the viral envelope. SARS-CoV-2 infection starts when the viral particles bind to host surface cellular receptors, namely ACE2, through the S protein, and posteriorly fuses to in order to gain entrance to the host cell.

The expressions "susceptibility to infection by severe acute respiratory syndrome coronavirus 2" or "susceptibility to infection by SARS-CoV-2" as used herein refer to the capacity of a subject to be infected by SARS-CoV-2 or not. In order to determine the general susceptibility for individuals to be infected, individuals with repeated exposure to high-risk infections situations were classified as being resistant, and therefore of low susceptibility to be infected by SARS-CoV-2. Examples of repeated exposure to high-risk infections were, without limitation, health workers working in COVID19 wards with repeated high-risk exposures to SARS-CoV-2 or children cohabiting with parents with confirmed COVID19 without isolation. In both cases, the individuals remained negative for COVID19, as determined by repeated polymerase chain reaction in nasopharyngeal exudate and/or by the negative IgG/IgM at least eight weeks after the exposure.

The use aspects disclosed in the present invention are further brought into practice by the methods describe hereafter.

Another aspect of the present invention relates to a method for the *in vitro* prediction of COVID19 severity in a subject, from here onwards the COVID19 prediction method of the invention, comprising:
a) Quantifying the level of activity of the ACE2 biomarker in a sample of saliva obtained from the subject; and
b) Comparing the activity level obtained in step a) with a reference value,
wherein a higher activity level value in the subject sample in comparison with the reference value is indicative of an increased severity of the disease in the subject.

Another aspect of the present invention relates to a method for the *in vitro* prediction of susceptibility to infection by SARS-CoV-2 in a subject, from here onwards the SARS-CoV-2 prediction method of the invention, comprising:
a) Quantifying the level of activity of the ACE2 biomarker in a sample of saliva obtained from the subject; and
b) Comparing the activity level obtained in step a) with a reference value,
wherein a higher activity level value in the subject sample in comparison with the reference value is indicative of increased susceptibility to SARS-CoV-2 infection by the subject.

The terms and expressions "COVID19", "COVID19 severity", "prediction of COVID19 severity", "SARS-CoV-2", "susceptibility to infection by SARS-CoV-2", "prediction of susceptibility to infection by SARS-CoV-2", "ACE2", "biomarker", "level of activity of the ACE2 biomarker", "sample" and "subject" have all been previously defined in relation to the use aspects of the invention and said definitions are equally valid for the present aspects and their embodiments.

The COVID19 prediction method of the invention and the SARS-CoV-2 prediction method of the invention are collective referred to as methods of the invention form here onwards.

In a preferred embodiment of the methods of the invention the subject is a mammal, preferably a human of any age and gender.

The expression "quantifying the level of activity of the ACE2 biomarker in a sample of saliva obtained from the subject" in the methods of the invention refers to the procedure wherein the capacity of the enzymatically active domain of ACE2, present in the saliva of a subject, has to hydrolyze or catalyze the cleavage of an appropriate target substrate is detected and measured. The skilled person in the art would be aware of methods and techniques which would allow the quantification of the activity of ACE2. One such method is the evaluation of the hydrolysis of the synthetic ACE2 substrate (7-methoxycoumarin-4-yl)acetyl - Alanine - Proline -Lysine - dinitrophenyl (Mca-APK-Dnp). In a preferred embodiment of the COVID19 prediction method of the invention or the SARS-CoV-2 prediction method of the invention the substrate is Mca-APK-Dnp. Mca-APK-Dnp is characterized by comprising the fluorescence molecule (7-methoxycoumarin-4-yl)acetyl and the quencher molecule dinitrophenyl which are bound to each other by three amino acids, alanine-Proline-Lysine, which constitute the target for hydrolysis by ACE2. The metabolization of Mca-APK-Dnp by ACE2 leads to the release of the fluorescence probe from the quencher molecule and therefore the emission of fluorescence, which is detectable and quantifiable by well-known fluorometric assays. (7-methoxycoumarin-4-yl)acetyl has an excitation range of approximately 260 to 360 nanometers (nm) of wavelength with a peak at 322 nm and a emission range of approximately 335 to 500 nm, with a peak at 381 nm. Therefore, in another preferred embodiment of the COVID19 prediction method of the invention or the SARS-CoV-2 prediction method of the invention the quantification in step a) is performed by fluorometric assays. In a further preferred embodiment, the quantification in step a) is performed by fluorometric assays and the substrate is Mca-APK-Dnp. Any probe containing Mca-APK-Dnp can be used in the methods of the invention. In yet a further preferred embodiment the quantification in step a) is performed by exciting the sample at a wavelength of between 280 to 340 nm and reading the emission of the sample at a wavelength of between 360 to 420 nm.

Step a) of the methods of the invention may require that the sample is treated before quantification is performed. Therefore, in a preferred embodiment of the methods of the invention the saliva sample is incubated in a buffer solution before step a) is performed. The term "buffer solution" as used herein refers to an aqueous solution consisting of a mixture of a weak acid and its conjugate base, or vice versa, whose pH changes very little when a small amount of strong acid or base is added to it. Examples of buffering agents which may find use in the methods of the invention are, without limitation, citric acid, acetic acid, monopotassium phosphate, N-Cyclohexyl-2-aminoethanesulfonic acid, borate, TAPS-[tris(hydroxymethyl)methylamino]propanesulfonic acid, Bicine - 2-(bis(2-hydroxyethyl)amino)acetic acid, tris(hydroxymethyl)aminomethane, potassium chloride, hydrochloride acid, sodium chloride, amongst others. The expert in the art will be well aware of the buffer solutions possibilities and which to choose from in order to perform the methods of the invention. Buffer solutions may contain other elements which while not part of the buffer solution find use in the realization of the methods of the invention. Examples of such agents are, without limitation, protease inhibitors, DNase inhibitors, RNase inhibitors, pH indicators, amongst others. Again, these agents are well known to the expert in the field who will be aware of the requirements for the present invention methods and will be able to determine which agents to use and their concentrations.

The methods of the invention can be performed in a single assay as well as in batch assays, wherein several assays are performed together. Therefore, in a preferred embodiment of the COVID19 prediction method of the invention or the SARS-CoV-2 prediction method of the invention, step a) is performed in a single or batch assay. The term "batch" as used herein refers to the realization of two or more assays of step a) of the methods of the invention, wherein the level of activity of ACE2 is analyzed for two or more samples of saliva form one or more subjects.

The quantification of the level of activity of the ACE2 biomarker can also be performed using the materials and procedures set forth in the document PCT/EP10/001770. Therefore, in a preferred embodiment of the methods of the invention, the quantification of the level of activity of the ACE2 biomarker in step a) is performed by using a probe, from here onwards the probe of the invention, which comprises a transition metal complex and a reactive component of general formula (X): 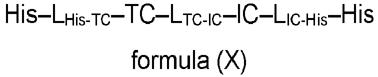 wherein His represents a histidine residue, TC represents an appropriate substrate for ACE2, IC represents an indicator component, and each of L_{His-TC}/L_{TC-IC} and L_{IC-His} independently represents optional linker components, wherein the reactive component is linked to the transition metal complex by the two histidine residues. In the present invention the components TC and IC of the probe can be represented by one unique complex substrate which contains both the hydrolyzing target for ACE2 as well as the indicator target, such as, but not limiting to, Mca-APK-Dnp. Therefore, in a preferred embodiment of the methods of the invention the probe of the invention has a reactive component of general formula (X1):

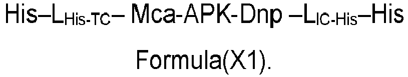

In another preferred embodiment of the methods of the invention the probe of the invention is further attached to an anchoring component according to formula (Y): 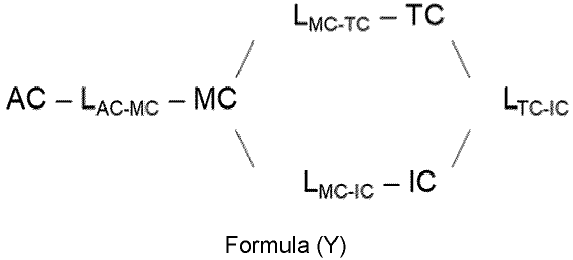 wherein AC represents an optional anchoring component, MC represents the transition metal complex, TC represents the test component, IC represents the indicator component, and L_{AC-MC}, L_{MC-TC}, L_{TC-IC} and L_{MC-IC} each independently represents an optional linker component between the respective components indicated by the subscripts, wherein it is preferred that L_{MC-TC} and L_{MC-IC} each independently comprise a histidine residue. In another preferred embodiment of the methods of the invention, the probe of the invention has a general formula (Y1):

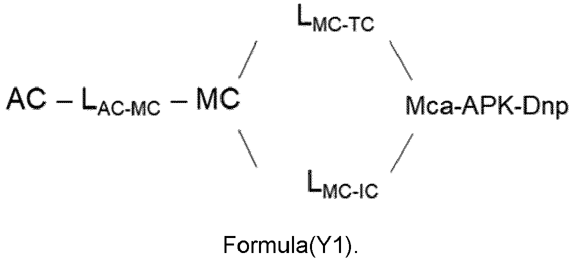

As used herein the term "linked" means that two components are connected with each other by means of at least one chemical bond, preferably by one, two, or three chemical bonds, and most preferred by exactly one chemical bond. A chemical bond indicates any chemical bond known in the art, such as, for example, an ionic bond, a covalent bond, including a single bond, a double bond, a triple bond, or another suitable multiple bond, and a hydrogen bond, and the like. Preferably, the chemical bond between the individual components is a covalent single bond. Preferably, the "link" or chemical bond between two components is a direct bond between the two components; but it may also comprise a suitable linker atom or molecule, if necessary. In some cases, one or both of the components to be linked will have to be activated in order to allow for the formation of a chemical bond or link. The procedures and means to be applied for such activation and formation of chemical bonds are standard knowledge of the field, and any skilled person will be aware of them.

Since the individual components are required to be linked (or chemically bonded) to each other as specified, the term "component" is used to indicate the respective parts of the probe compound which are characterised by their respective function as determined in the following. Herein, the term "component" is meant to refer to a specified part or moiety of a molecule, which is identified by its function within this molecule. For example, the term "indicator component" refers to a part or moiety of the molecule, which comprises a signal-generating function, e.g. a fluorescence dye or the like, that allows for indicating the location of the probe compound. Similarly, the term "test component" refers to a part or moiety of the molecule, which comprises a substrate or metabolite, thus being the site of enzymatic interaction with the probe compound. The respective components are linked by chemical bonds to form the molecule, i.e. the probe compound. It should be noted that, in the context of this invention, the term "component" is used with the meaning of "part" or "moiety" of one compound or molecule, and not to refer to individual members of a multi -molecule system. The terms "component", "part" and "moiety" may be used alternatively with this meaning. However, for reasons of consistency, the term "component" will also be used to refer to the respective molecules before they are reacted to form the respective part (component) of the probe compound, or after they are released from the probe compound, respectively. A person skilled in the art will readily understand the exact nature of the respective molecules, as well as their contribution to the probe compound.

The term "test component" as used herein refers to the part or component of the probe compound which comprises a substrate or metabolite appropriate, as previously defined, to be hydrolysed or cleaved by the enzymatic activity of ACE2. The substrate or metabolite is comprised in the test component in such a manner that its characteristic structure and/or functional groups necessary for interaction with the active site of an enzyme are maintained within the test component. Therefore, the substrate or metabolite is preferably linked to the other components of the probe compound at positions of the substrate or metabolite molecule, which are not involved in enzyme-substrate interaction. Optionally, a spacer or linker moiety can be used to provide a suitable binding position on the test component, which allows for an unimpeded enzyme interaction. In this case, the term "test component" refers to the component comprising both the substrate and the spacer moiety. Moreover, the test component can be preferably functionalised with a linker component or moiety suitable for binding to the transition metal complex. Such functionalisation is especially advantageous for test components comprising substrates, which do not readily form coordination bonds. In a preferred embodiment, the test component is functionalised with a histidine molecule or residue (sometimes referred to as a "His-tag"). The term "indicator component" is used to indicate a molecule which can generate a signal, thus indicating the location of the probe molecule. That signal can either be produced directly, for example by adsorbance or fluorescence, or can be generated in further treatments with detection reagents, for example in the form of an optical or radioactive signal. Preferably, the indicator component comprises a dye, further preferred a fluorescence dye. The term "fluorescence dye" indicates a molecule showing fluorescence upon irradiation with a suitable light source.

The test component is linked to the indicator component to form the reactive component. Preferably, the test component is linked to the indicator component by at least one covalent chemical bond, further preferred by one, two, or three covalent bonds, and still further preferred by exactly one covalent bond. A preferred example of such a covalent bond is a carbon-oxygen single bond, which can be formed between the test component and the indicator component by various chemical reactions, such as, for example, a condensation reaction, an addition reaction, an oxidation reaction, and the like. A preferred example is a condensation reaction between a carboxylic acid and an alcohol, or between two alcohols, or an addition reaction wherein the oxygen atom of an alcohol function is added to an aliphatic or aromatic carbon atom, or the like. It should be noted that such bond forming reaction is not restricted to the examples given above, and that there is no prejudice regarding which individual function should be present in the respective molecules to become the respective components, etc. A person skilled in the art will immediately know which combinations of functional groups will be required to form a corresponding chemical bond between the individual molecules to become the respective components of the probe compound, and also which starting materials and conditions etc. are required to form the desired chemical bond between the components. Another preferred covalent bond is a carbon- nitrogen single bond. Preferably, the carbon-nitrogen single bond is part of a quaternary amine function (quaternary ammonium function) comprised in the link between test component and indicator component. The link or bond between the test component and the indicator component may also be formed between the test component and a linker moiety previously attached to the indicator component, or vice versa. An additional linker moiety has the advantage to ensure an identical and reproducible binding to the test component, which allows for the ready use in parallel synthesis or the like. In a preferred embodiment, the indicator component comprises an amino butyric acid linker moiety, and a bond or link to the test component is formed using the carboxylic acid function of this linker moiety. Preferably, the link between indicator component and test component comprises a linker moiety comprising an amino butyric acid linker residue attached to the indicator component, the carboxylic acid function of which is linked to another linker moiety comprising a quaternary amine function, which in turn is linked to the test component. Such linker moiety can be formed, for example, by reacting an indicator component, e.g. a fluorescence dye, first with 4-amino-3-butanoate and then with N,N-dimethylethanolamine. Linking with the test component under formation of a quaternary amine can then be obtained by reacting the so- prepared indicator component having a linker moiety comprising an amino butyric acid residue, the carboxylic acid function of which is esterified by N, N- dimethylethanolamine, with an iodine-containing test component in the presence of 1, 8-bis- (dimethylamino) - naphthalene, or a similar method.

The reactive component comprising the test component and the indicator component, which are linked to each other, optionally by a linker moiety or molecule, is in turn linked to the transition metal complex. Preferably, the reactive component is linked to the transition metal complex by two, three or four coordination bonds, and further preferred by exactly two coordination bonds. That means that at least one atom of the reactive component is a direct ligand atom of the transition metal atom of the transition metal complex, thus being part of the immediate coordination sphere of the central transition metal atom of the transition metal complex. The term "at least one atom of the reactive component" also includes an atom of a linker moiety, which can optionally be attached to either the test component or the indicator component. For example, in a preferred embodiment, a histidine molecule is attached as a linker moiety (His-tag) to the reactive component. In this case, the "at least one atom of the reactive component" can also indicate an atom of the His-tag, preferably a nitrogen atom of the imidazole ring system. Preferably, the reactive component is linked to the transition metal complex by two coordination bonds, wherein one ligand atom is an atom of the test component and the other ligand atom is an atom of the indicator component. In a preferred embodiment, the reactive component comprises a His-tag linked to the test component and a His-tag linked to the indicator component. In this case, the reactive component is linked to the transition metal complex by two coordination bonds, wherein each bond includes one atom of one of the respective His- tags.

A preferred coordination bond between the reactive component and the transition metal complex is a M-O-R-bond, wherein M indicates the transition metal atom and O-R indicates an oxygen atom or function of a molecule R constituting the reactive component. Preferred examples of suitable oxygen functions are an alcoholate function (R-O'), a carboxylate function (RCOO"), a peroxide function (R-O-O"), or the like. Another preferred coordination bond between the reactive component and the transition metal complex is a M-N-R-bond, wherein M indicates the transition metal atom and N-R indicates a nitrogen atom or function of a molecule R constituting the reactive component. An example for such nitrogen function is an aliphatic amine function, including a primary, secondary and tertiary amine function. The nitrogen atom can also be part of an aromatic or (partially)saturated ring system, such as, for example, a pyrrol, imidazole, diazole or triazole ring, or the like. A specially preferred coordination bond is a coordination bond comprising a nitrogen atom of an imidazole ring, which may be part of a histidine moiety or His-tag. Another preferred coordination bond is a M-S-R or a M-C-R single bond, wherein M indicates the transition metal atom and S and C, respectively, indicate a sulphur or carbon function of a molecule R constituting the reactive component. It should be noted that the coordination bond between reactive component and transition metal complex is not restricted to the examples given above, and that there is no prejudice regarding which individual function should be present in the respective molecules to form the desired coordination bond, etc. A person skilled in the art will immediately know which functional groups will be required to form a corresponding coordination bond, and also which starting materials and conditions etc., are required.

The transition metal complex preferably comprises at least one transition metal atom and at least one ligand molecule, wherein at least one coordination bond is formed between the ligand molecule and the transition metal atom. The at least one ligand molecule preferably comprises one or more atoms or functions which can form a coordination bond with a transition metal atom. Preferred examples for atoms or function are an oxygen atom, a nitrogen atom, a phosphorous atom, a sulphur atom, or the like. These atom or functions all can form a coordination bond with a transition metal atom and are the same as exemplified above. Preferably, a ligand molecule comprises more than one atom or function that can form a coordination bond with a transition metal atom, further preferred two to eight of such atoms or functions, still further preferred three to five of such atoms or functions, and most preferred four or five of such atoms of functions. In a preferred embodiment of the present invention, the transition metal complex comprises exactly one transition metal atom and exactly one ligand molecule comprising more than one atom or function that can form a coordination bond with a transition metal atom. Herein, the term "transition metal atom" is used to indicate the central transition metal atom of the transition metal complex, which is linked to both the, ligand molecule (s) and the reactive component by coordination bonds as defined above. Examples of suitable transition metal atoms are Ti, V, Mn, Fe, Co, Ni, Cu, Zn, Mo, W, Pt, Au, or the like. Preferred examples are Co, Ni, and Cu. The transition metal atom means any transition metal atom irrespective of its oxidation state. The term transition metal ion is also used for a transition metal atom that carries a net charge, which is sometimes referred to as a "transition metal ion" in the art, wherein a formal charge or oxidation state is assigned to the transition metal atom or ion. Preferred oxidation states of transition metal atoms of the present invention are from 0 to +4, preferably +2 to +3, and especially preferred +2. Preferred examples of transition metal atoms are Co(II), Ni(II), and Cu(II). Accordingly, the coordinating atoms or functions of the ligand molecule may be assigned with a formal charge, wherein preferred charges range from 0 to -2, wherein a charge of 0 or -1 is especially preferred. As used herein, the term "ligand molecule" refers to a molecule that comprises at least one atom or function which forms a direct coordination bond to the central atom of a transition metal complex, preferably one, two, three, four, five, six, seven, or eight such atoms or functions. It should be noted that the coordination bond between ligand molecule and transition metal atom is not restricted to the examples given above, and that there is no prejudice regarding which individual function should be present in the ligand molecule to form the desired coordination bond, etc. A person skilled in the art will immediately know which functional groups will be required to form a corresponding coordination bond, and also which starting materials and conditions etc. are required.

Once the level of activity of the ACE2 biomarker has been quantified, the value obtained must be compared to a reference value in order to determine the outcome of the method. The expression "Comparing the activity level obtained in step a) with a reference value" as used herein refers then to the procedure of weighing against, examine, contrast or verify the values obtained for the level of activity of the ACE2 biomarker, and equate or verify the values obtained in step a) with the reference values. The term "reference value" as used herein refers to the average value of the level of activity of the ACE2 biomarker in saliva obtained in a pool of individuals which are resistant to infection by SARS-CoV-2 and do not suffer from COVID19 and/or a pool of subjects which are susceptible to infection by SARS-CoV-2.

Therefore, in the COVID19 prediction method of the invention the reference value corresponds to the average level of activity of the ACE2 biomarker in saliva of a pool of individuals which are susceptible to COVID19 as previously define. Therefore, a higher level of activity in the subject sample in comparison to the reference value is indicative of increase severity of the disease in the subject. Consequently, a lower level of activity in the subject sample in comparison to the reference value is indicative of reduced severity of disease in the subject.

In the SARS-CoV-2 prediction method of the invention the reference value corresponds the average level of activity of the ACE2 biomarker in saliva of a pool of individuals which are resistant to COVID19 as previously defined. As such, a higher level of activity in the subject sample in comparison with the reference value is indicative of increased susceptibility to SARS-CoV-2 infection by the subject.

The term "higher" refers to a greater or larger level of activity of the ACE2 biomarker obtained in a saliva sample form a subject which is either resistant or susceptible to SARS-CoV-2 infection when these values are compared with the corresponding reference values as previously defined for the methods of the invention. In particular, "higher" level of activity of the ACE2 biomarker is understood to be when the activity level is at least 0.1-fold, 0.5-fold, 1-fold, 5-fold, 10-fold, 10-fold, 20-fold, 30-fold, 40-fold, 50-fold, 60-fold, 70-fold, 80-fold, 90-fold, 100-fold or even more with respect to the reference values previously defined for the methods of the invention.

Likewise, the term "lower" refers to a reduced or smaller level of activity of the ACE2 biomarker obtained in a saliva sample form a subject which is susceptible to SARS-CoV-2 infection when these values are compared with the corresponding reference values as previously defined for the SARS-CoV-2 prediction method of the invention. In particular, "lower" level of activity of the ACE2 biomarker is understood to be when the activity level is at least 0.1-fold, 0.5-fold, 1-fold, 5-fold, 10-fold, 10-fold, 20-fold, 30-fold, 40-fold, 50-fold, 60-fold, 70-fold, 80-fold, 90-fold, 100-fold or even less with respect to the reference values previously defined for the methods of the invention.

The terms "increased severity" and "reduced severity" as used herein refers to the higher and lower probability of the subject developing severe COVID19 symptoms (previously defined). As those skilled in the art will understand, such an assessment based on probability, although preferred, may not normally be correct for 100% of the subjects to be assessed. The term, however, requires that a statistically significant proportion of the subjects can be identified as developing or not a severe case of COVID19. The number that is statistically significant can be established by a person skilled in the art by using different statistical tools, for example, but not limited to, determination of confidence intervals, determination of P-significance value, Student's test or Fisher's discriminant functions, Mann Whitney non-parametric measures, Spearman's correlation, logistic regression, linear regression, area under the ROC curve (AUC). Preferably, the confidence intervals are at least 90%, at least 95%, at least 97%, at least 98% or at least 99%. Preferably, the p-value is less than 0.1, less than 0.05, less than 0.01, less than 0.005 or less than 0.0001. Preferably, the present invention allows to correctly detect the severity of COVID19 or susceptibility of being infected by SARS-CoV-2 differentially in at least 70%, more preferably in at least 80%, much more preferably in at least 90% of the subjects of a given group or population tested.

The expression "increased susceptibility to SARS-CoV-2 infection" as used herein refers to the higher probability of the subject being infected by SARS-CoV-2 and developing COVID19. As those skilled in the art will understand, such an assessment based on probability, although preferred, may not normally be correct for 100% of the subjects to be assessed. The term, however, requires that a statistically significant proportion of the subjects can be identified as having increased susceptibility of being infected by SRAS-CoV-2. The number that is statistically significant can be established by a person skilled in the art by using different statistical tools, for example, but not limited to, determination of confidence intervals, determination of P-significance value, Student's test or Fisher's discriminant functions, Mann Whitney non-parametric measures, Spearman's correlation, logistic regression, linear regression, area under the ROC curve (AUC). Preferably, the confidence intervals are at least 90%, at least 95%, at least 97%, at least 98% or at least 99%. Preferably, the p-value is less than 0.1, less than 0.05, less than 0.01, less than 0.005 or less than 0.0001. Preferably, the present invention allows to correctly detect the severity of COVID19 or susceptibility of being infected by SARS-CoV-2 differentially in at least 70%, more preferably in at least 80%, much more preferably in at least 90% of the subjects of a given group or population tested.

The methods of the invention can be set forth as kits wherein all the materials required for their application are provided. As such, another aspect of the invention relates to a kit which comprises substrates, probes such as the probe of the invention and/or markers specific for the quantification of the activity level of ACE2 biomarker in a saliva sample isolated from a subject, from here onwards the kit of the invention. The kit may further include other components that find use in the subject invention, e.g., buffers, delivery vehicles, substrate, probes and/or markers supports, positive and/or negative controls components optionally wherein such controls comprise substrates, probes and/or markers, containers with solutions or empty containers that encounter use in the practicing of the subject invention. The various components of the kit may be present in separate containers or certain compatible components may be combined into a single container, as desired. In addition to the above components, the subject kits will further include instructions for practicing the subject invention. These instructions may be present in the subject kits in a variety of forms, one or more of which may be present in the kit. One form in which these instructions may be present is as printed information on a suitable medium or substrate, e.g., a piece or pieces of paper on which the information is printed, in the packaging of the kit, in a package insert, etc. Yet another means would be a computer readable medium, e.g. a CD, an USB, etc., on which the information has been recorded. Yet another means that may be present is a website address or a QR code which may be used via the internet to access the information at a removed site. Any convenient means may be present in the kits.

The kit of the invention finds use in the prediction of COVID19 severity and prediction of susceptibility to infection by SARS-CoV-2. Thus, another aspect of the present invention relates to the *in vitro* use of the kit of the invention for the prediction of COVID19 severity in a subject, from here onwards the COVID19 severity kit use of the invention. Another aspect of the present invention relates to the *in vitro* use of the kit of the invention for the prediction of susceptibility to infection by SARS-CoV-2 in a subject, form here onwards the SARS-CoV-2 infection kit use of the invention.

All the term and expressions previously defined in relation with other aspects of the present invention are equally valid for the current aspects and their embodiments.

In a preferred embodiment of the COVID19 severity kit use of the invention and of the SARS-CoV-2 infection kit use of the invention, the subject is a mammal, preferably a human of any age and gender. In another preferred embodiment the substrate is methoxycoumarin-4-yl)acetyl - Alanine - Proline -Lysine - dinitrophenyl (Mca-APK-Dnp). In another preferred embodiment the substrate corresponds to the probe of the invention according to general formula (Y) or (Y1), preferably wherein the reactive component of the probe of the invention comprises the formula (X1).

### DESCRIPTION OF THE DRAWINGS

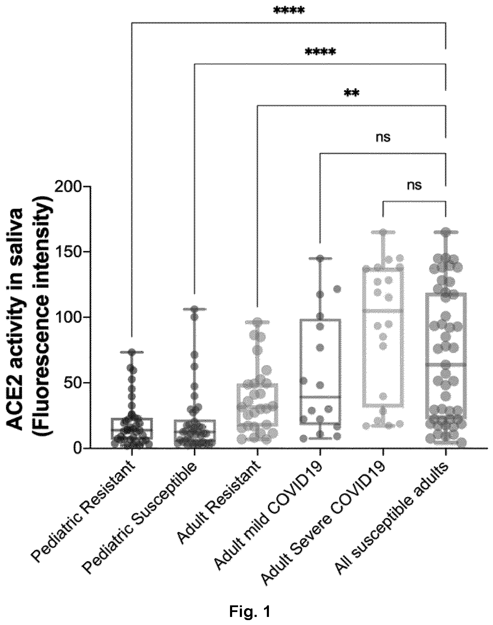

**Fig. 1****. Comparison of ACE2 activity in saliva according to susceptibility and disease severity in adult and pediatric population.** The graph is a box and whiskers plot showing all values as points. The groups were compared by one-way ANOVA followed by Tukey test. The adult resistant group was the reference category for the multiple comparisons. ** denotes a p value < 0.01 and > 0.001, **** denotes a p value < 0.0001.

### Examples

### Example 1

### Study design and setting

Data from children recruited at Hospital Universitario La Paz and adults recruited at Hospital Universitario Ramon y Cajal were analysed. Children who were positive for SARS-CoV-2 IgG and had shown mild or no symptoms suggestive or SARS-CoV-2 infection were classified as "susceptible", and as "resistant" those with repeated high-risk exposures to SARS-Cov-2, specifically, cohabiting and not isolating with parents with confirmed COVID19, but who remained IgG negative at least after eight weeks after the exposure. Adults were considered as "susceptible" when they had positive IgG for SARS-CoV-2 or previous COVID19 confirmed by polymerase chain reaction (PCR) in nasopharyngeal exudate. Non-susceptible adults were healthy healthcare workers who had been on duty for at least three months in COVID19 wards or intensive care units and reported at least three high-risk exposures to SARS-CoV-2, without having experienced symptoms suggestive of SARS-CoV-2 infection, persistently negative PCR SARS-CoV-2 testing, and absence of SARS-CoV-2 IgM and IgG in plasma. The most frequent exposure was largely unprotected exposure to aerosol-generating procedures or patient secretions and close contact without face masks with other confirmed cases of COVID-19. We measured SARS-CoV-2 antibodies by indirect chemiluminescence immunoassay (Vircell, Granada, Spain). Participants with mild disease were those without a need of supplemental oxygen and asymptomatic after one week of diagnosis. Severe disease was defined as the presence of bilateral radiologic infiltrates or opacities and clinical assessment requiring supplemental oxygen.

### ACE-2 activity measurement and statistical analysis

Non-induced saliva samples were collected in sterile tubes and cryopreserved at - 80°C. After thawing, ACE2 activity was measured in batch through a fluorometric assay, using a synthetic ACE2-specific substrate (Mca-APK(Dnp) (Reactomix S.L., Granada, Spain), or any other probe containing Mca-APK-Dnp, that is metabolized to a fluorescent compound in the presence of a functional enzyme. Prior to the assay a substrate stock solution in dimethylsulfoxide was prepared (1 mg per ml). Cultures of saliva samples were incubated with the buffer solution (150nM Tris-HCI pH 7.5, 200nM NaCl, 10 uM ZnCI2, protease inhibitor) and the substrate, at the following portions: 10 parts of saliva, 99 parts of buffer solution and 1 part of substrate, respectively). After incubation at room temperature for 16 h, we quantified each culture's fluorescence using Varioskan Lux (Thermo), a fluorescence reader with an excitation of 320 nm and an emission of 420 nm. Measurements in duplicate showed a coefficient of variation <10%. According to this technique, increased fluorescence indicates increased ACE2 activity.

We used the one-way ANOVA followed by the Tukey test to correct for multiple comparisons and logistic regression to establish the association between susceptibility to SARS-CoV-2 and ACE2 activity after controlling for the potential confounding effect of sex. We used Stata version 16.0 (StataCorp LP, College Station, Texas) for the statistical analysis and Prism version 9.0 (GraphPad, La Jolla, California) for figure generation.

### Results

74 adults, of which 47 (64%) were susceptible and 27 (36%) were resistant, and 79 children, of which 41 (52%) were susceptible and 38 (48%) were resistant, were included. The age range was 5 to 92 years. Among the 47 susceptible adults, 20 (43%) had severe disease, 16 (34%) mild disease, and 11 (23%) were people who had asymptomatic COVID-19 and had positive IgG with negative PCR test at the time of inclusion in the study. Table 1 shows the baseline characteristics of the sample population.

**Table 1. Population baseline characteristics.**

| | **Adults (n = 74)** | **Pediatrics (n = 79)** |
|---|---|---|
| Age, median (p25-p75) | 51 (40-60) | 10 (8-13) |

| Gender, n (%) | | |
|---|---|---|
| Female | 44 (59%) | 38 (49%) |

| Susceptibility, n (%) | | |
|---|---|---|
| Susceptible | 47 (64%) | 41 (52%) |
| Resistant | 27 (36%) | 38 (48%) |

| Comorbidities, n (%) | | |
|---|---|---|
| Hypertension | 17 (23%) | 0 (0%) |
| Diabetes | 2 (3%) | 0 (0%) |
| Obesity | 8 (11%) | 0 (0%) |
| Lung diseases | 6 (8%) | 0 (0%) |
| Cardiac disease | 8 (11%) | 0 (0%) |

| Days from symptom onset to hospitalization, median (IQR) | | |
|---|---|---|
| Mild COVID19 | 7 (4 - 10) | * |
| Severe COVID19 | 4 (2-7) | * |

| | | |
|---|---|---|
| * No pediatric participants required hospital admission. All had asymptomatic/mild disease. Abbreviations: p25-p75, 25^{th} percentile - 75^{th} percentile. | | |

Figure 1 shows the difference in ACE2 activity in saliva between susceptible and resistant individuals, with a much smaller difference among the pediatric population median fluorescence (12 vs. 14 FU units, p= 0.561) than in adults (64 vs. 31 FU units, p = 0.0116). Resistant adults have significantly lower ACE2 activity in saliva than susceptible adults and non-significant higher values than susceptible and resistant children. ACE2 activity is similar in the susceptible and resistant pediatric population (p=0.527). In contrast, we observe an increase in activity as the disease's severity increases among the adult population (mild disease vs. severe disease, 39 vs. 105 FU, p=0.039; severe disease vs. resistant, 105 vs. 31 FU, p<0.001).

The logistic regression model yielded similar estimates in the unadjusted model and after controlling by sex (Table 2). We observed significant differences between the susceptible and resistant adult population in the adjusted model (p=0.008) but not in the pediatric population (p=0.790). Table 3 shows the results of the adjusted multinomial model by disease severity in adults.

**Table 2. Unadjusted and adjusted logistic regression models estimates.**

| | **Coefficient** | **95% CI** | **p** |
|---|---|---|---|
| Adults | | | |
| *Crude model* | | | |
| Susceptible | Ref. | - | - |
| Resistant | -0.020 | -0.034, -0.006 | 0.005 |

| *Adjusted model** | | | |
|---|---|---|---|
| Susceptible | Ref. | - | - |
| Resistant | -0.019 | -0.033, -0.005 | 0.008 |

| Pediatrics | | | |
|---|---|---|---|
| *Crude model* | | | |
| Susceptible | Ref. | | - |
| Resistant | -0.002 | -0.023, 0.019 | 0.849 |

| *Adjusted model** | | | |
|---|---|---|---|
| Susceptible | Ref. | | - |
| Resistant | -0.003 | -0.024, 0.018 | 0.790 |

| | | | |
|---|---|---|---|
| *Model adjusted for sex | | | |

**Table 3. Multinomial logistic regression model estimates for saliva ACE2 expression by disease severity in adult population.**

| **Disease Severity** | **Coefficient** | **95% CI** | **p** |
|---|---|---|---|
| Resistant | Ref. | - | - |
| Mild Disease * | 0.013 | -0.004, 0.031 | 0.128 |
| Severe Disease * | 0.001 | 0.013, 0.048 | 0.001 |

| | | | |
|---|---|---|---|
| *Model adjusted for sex | | | |

## Claims

1. A use of the level of activity of the angiotensin-converting enzyme 2 (ACE2) biomarker in a saliva sample isolated from a subject for the *in vitro* prediction of coronavirus disease 2019 (COVID19) severity.

2. A use of the level of activity of the ACE2 biomarker in a saliva sample isolated from a subject for the *in vitro* prediction of susceptibility to infection by severe acute respiratory syndrome coronavirus 2 (SARS-CoV-2).

3. The use according to any of claims 1 or 2, wherein the subject is a mammal, preferably a human.

4. A method for the *in vitro* prediction of COVID19 severity in a subject comprising:
a) Quantifying the level of activity of the ACE2 biomarker in a sample of saliva obtained from the subject; and
b) Comparing the level of activity obtained in step a) with a reference value,
wherein a higher level of activity in the subject sample in comparison with the reference value is indicative of an increased severity of the disease in the subject.

5. A method for the *in vitro* prediction of susceptibility to infection by SARS-CoV-2 in a subject comprising:
a) Quantifying the level of activity of the ACE2 biomarker in a sample of saliva obtained from the subject; and
b) Comparing the level of activity obtained in step a) with a reference value,
wherein a higher level of activity in the subject sample in comparison with the reference value is indicative of increased susceptibility to SARS-CoV-2 infection by the subject.

6. The method according to claims 4 or 5 wherein the quantification in step a) is performed by fluorometric assay.

7. The method according to claim 6 wherein the technique is a fluorometric assay and the substrate used in said assay is (7-methoxycoumarin-4-yl)acetyl - Alanine - Proline -Lysine - dinitrophenyl (Mca-APK-Dnp).

8. The method according to any one of claims 4 to 7 wherein the quantification in step a) is performed using a probe which comprises a transition metal complex and a reactive component of general formula (X): 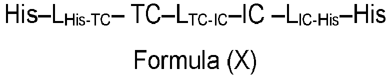 wherein His represents a histidine residue, TC represents an appropriate substrate for ACE2, IC represents an indicator component, and each of L_{His-TC}/L_{TC-IC} and L_{IC-His} independently represents optional linker components, wherein the reactive component is linked to the transition metal complex by the two histidine residues.

9. The method according to claim 8 wherein the reactive component has the general formula (X1):

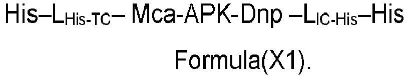

10. The method according to any one of claims 4 to 9 wherein the subject is a mammal, preferably a human.

11. *In vitro* use of a kit which comprises substrates, probes and/or markers specific for the quantification of the activity level of ACE2 biomarker in a saliva sample isolated from a subject, for the prediction of COVID19 severity in a subject.

12. *In vitro* use of a kit which comprises substrates, probes and/or markers specific for the quantification of the activity level of ACE2 biomarker in a saliva sample isolated from a subject, for the prediction of susceptibility to infection by SARS-CoV-2 in a subject.

13. The use according to claims 11 or 12 wherein the subject is a mammal, preferably a human.

14. The use according to any one of claims 11 to 13 wherein the substrate is (7-methoxycoumarin-4-yl)acetyl - Alanine - Proline -Lysine - dinitrophenyl (Mca-APK-Dnp).

15. The use according to any one of claims 11 to 14 wherein the probe comprises a transition metal complex and a reactive component of general formula (X): 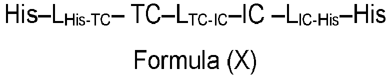 wherein His represents a histidine residue, TC represents an appropriate substrate for ACE2, IC represents an indicator component, and each of L_{His-TC}/L_{TC-IC} and l_{IC-His} independently represents optional linker components, wherein the reactive component is linked to the transition metal complex by the two histidine residues, preferably wherein the reactive component has the general formula (X1):
